# EUROPEAN PATENT APPLICATION

(11) **EP 4 700 790 A1**
(43) Date of publication of application: **25.02.2026**
(21) Application number: 25192079.9
(22) Date of filing: 28.07.2025
(51) Int. Cl.: G16H 50/20, A61B 5/00

(54) **SYSTEMS AND METHODS FOR ADDING INTERPRETABILITY TO AND ASSESSING BIAS OF AN ECG ANALYSIS MODEL**

(30) Priority: 21.08.2024 GR 20240100584; 27.08.2024 US 202418817081
(71) Applicant: GE Precision Healthcare LLC, Waukesha, WI 53188 (US)
(72) Inventor: KARGIANTOULAKIS, Emmanouil, Milwaukee, 53223 (US)
(74) Representative: Kilburn & Strode LLP

(57) **Abstract**

Systems and methods for adding interpretability to and assessing bias of an ECG analysis model are herein provided. In one example, a method comprises: obtaining a diagnostic output from an Al-based ECG analysis model on an ECG dataset (302); extracting interpretable criteria from the ECG dataset for a target of the diagnostic output of the Al-based ECG analysis model to predict an output for the ECG dataset based on the extracted criteria; determining one or more characteristics of the extracted criteria (304); assessing the output from the ECG analysis model for bias based on a comparison between the output of the ECG analysis model and the predicted output (404); and outputting the one or more characteristics and the bias assessment to a user device (362).

## Description

### CROSS REFERENCE TO RELATED APPLICATIONS

The present application claims priority to Greek Patent Application No. 20240100584, filed on August 21, 2024. The entire contents of the above-listed application is hereby incorporated by reference for all purposes.

### FIELD

Embodiments of the subject matter disclosed herein relate to ECG analysis, and more particularly to systems and methods for adding interpretability to and assessing bias of an ECG analysis model.

### BACKGROUND

An ECG is a graphic representation of electrical activity of the heart, and is generally represented as a waveform. Traditional, rules-based ECG analysis models may receive an ECG, determine a set of features (e.g., findings) of the ECG, compare the set of features to respective criteria of a set of rules, determine a diagnosis based on the criteria met by the features of the ECG, and generate a diagnostic interpretation result of the ECG. A physician may review the ECG and/or the diagnostic interpretation result to assess the cardiac activity of a patient.

However, artificial intelligence (AI)-based ECG analysis models, such as neural networks, do not utilize the same sets of features and criteria in a predictable manner. Thus, such AI-based ECG analysis models often lack interpretability and are subject to confounding bias, limiting their acceptability and usage in clinical environments.

### BRIEF DESCRIPTION

In one example, a method comprises: obtaining a diagnostic output from an AI-based ECG analysis model on an ECG dataset, extracting interpretable criteria from the ECG dataset for a target of the diagnostic output to predict the diagnostic output from the AI-based ECG analysis model; assessing for bias in the AI-based ECG analysis model to evaluate the fitness of the AI-based ECG analysis model for deployment; and outputting the interpretable criteria and the bias assessment to a user device. By outputting the interpretable criteria that explain the diagnostic output of the AI-based ECG analysis model and the bias assessment, a user can more readily understand reasoning for the diagnostic output.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, wherein below:
FIG. 1 is a diagram of an example system for electrocardiogram (ECG) interpretation;
FIG. 2 is a diagram of example components of a device of the example system shown in FIG. 1;
FIG. 3A is a flowchart illustrating a method for adding interpretability for an AI-based ECG analysis model for an ECG dataset without ground truth;
FIG. 3B is a flowchart illustrating a method for assessing the AI-based ECG analysis model for bias;
FIG. 4 is a flowchart illustrating a method for assessing bias of an AI-based ECG analysis model;
FIG. 5 is a flowchart illustrating a method for extracting, using an AI model, criteria for adding interpretability to an AI-based ECG analysis model;
FIG. 6 is a diagram of example extracted criteria;
FIG. 7 is a graph of example prediction scores; and
FIG. 8 is a graph of example determined diagnoses.

### DETAILED DESCRIPTION

The following description relates to various embodiments of electrocardiogram (ECG) interpretation. In particular, systems and methods for adding interpretability and assessing bias of an artificial intelligence (AI)-based ECG analysis model, such as a machine learning model, neural network, or the like, are provided. Traditional, rules-based ECG analysis models may use ECG features and fully interpretable criteria developed by subject matter experts. Thus, the rules-based model may identify specific features and criteria in order to output a diagnosis or diagnostic interpretation.

In contrast, AI-based ECG analysis models lack this interpretability. Specifically, an AI-based ECG analysis model may output a diagnosis or diagnostic interpretation that is not fully explainable to a clinician (e.g., a physician or technician).

Additionally, AI-based ECG analysis models are often biased. Specifically, the training of the models can result in the model being biased towards certain outputs over others in the context of the same inputs. This lack of interpretability and instance of bias in ECG analysis models limits their usability in clinical settings as clinicians are less likely to trust the outputs when they cannot interpret why the output was produced.

Thus, systems and methods for adding interpretability and assessing bias of AI-based ECG analysis models is herein presented. By extracting fully-interpretable criteria on the ECG dataset for which the AI-based ECG analysis model is deployed, specifically for a target of the diagnostic output of the AI-based ECG analysis model, the output of the model can be predicted. Thus, the interpretable criteria may explain the diagnostic output from the AI-based ECG analysis model, thereby adding interpretability to the model. Further, based on the extracted criteria, bias in the outputs can be assessed for. Interpretability and bias assessment may be presented to a user (e.g., a clinician, physician, care provider, ECG technician, etc.), who may thus be able to more dependably assess the outputs of the ECG analysis model, thereby increasing the usability of such models in clinical settings.

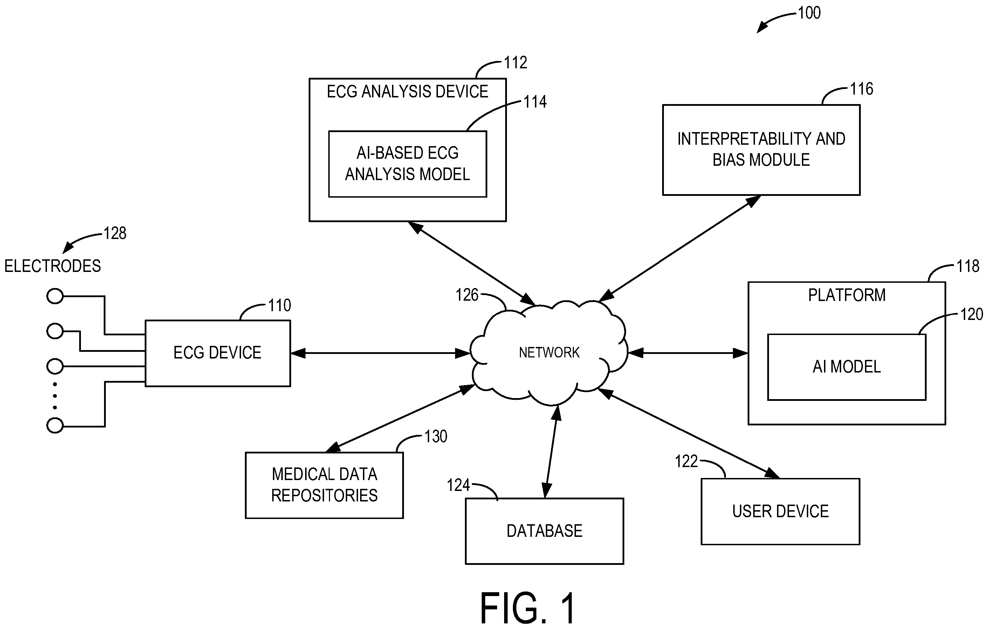

The systems and methods herein disclosed will now be described, by way of example, with reference to the figures, wherein FIG. 1 shows a diagram of an example ECG analysis system,

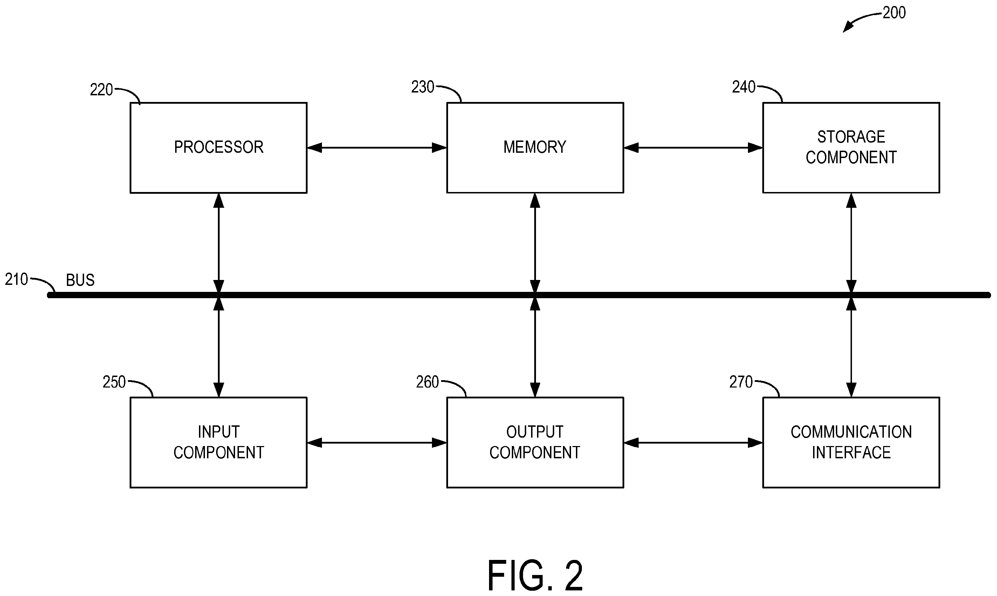

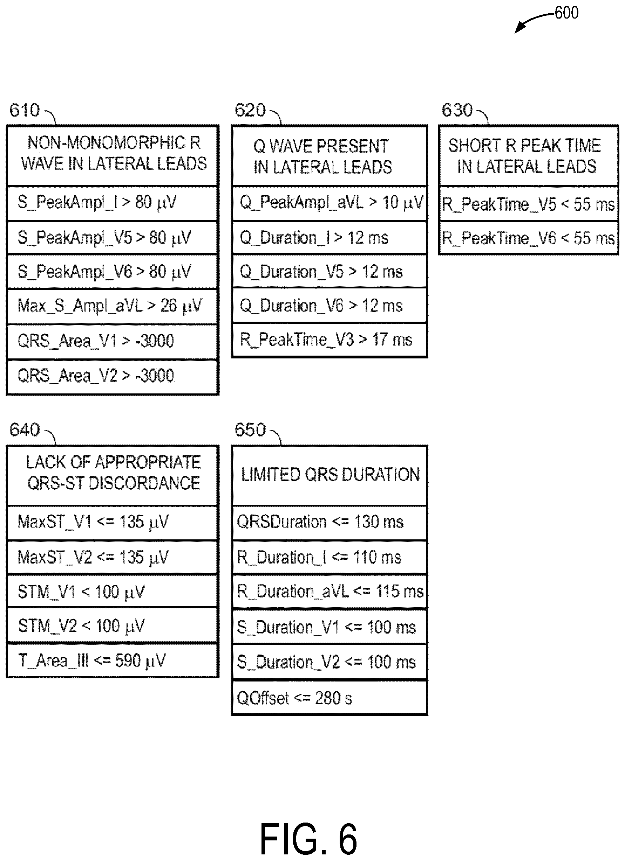

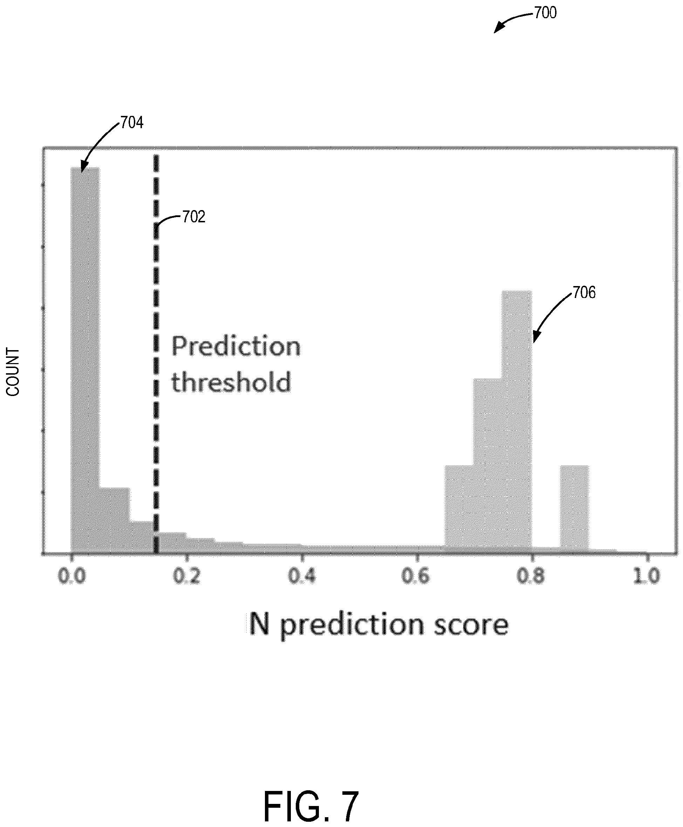

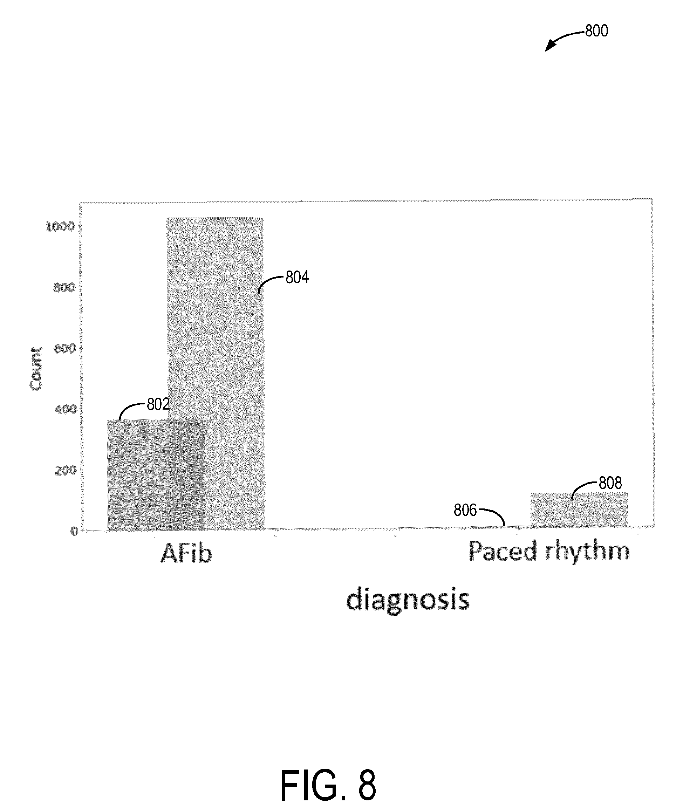

FIG. 2 shows a diagram of example components of a device of the example system shown in FIG. 2, FIGS. 3-5 show flowcharts illustrating methods for adding interpretability to an ECG analysis model, assessing bias of the ECG analysis model, and extracting criteria for determining a diagnosis by the ECG analysis model, FIG. 6 shows a diagram of example extracted criteria, FIG. 7 shows a graph of prediction scores, and FIG. 8 shows a graph depicting bias of an ECG analysis model.

Starting with FIG. 1, a diagram of an example ECG analysis system 100 is shown. The ECG analysis system 100 may be configured to extract interpretable criteria on a diagnostic output of an AI-based ECG analysis model and add interpretability to an ECG analysis model. As shown in FIG. 1, the system 100 may include an ECG device 110, a plurality of electrodes 128, an ECG analysis device 112, an AI-based ECG analysis model 114, an interpretability and bias module 116, a platform 118, an AI model 120, a user device 122, a database 124, one or more medical data repositories 130, and a network 126.

ECG device 110 may be configured to generate an ECG tracing of a patient. For example, the ECG device 110 may be a stand-alone ECG device, a portable ECG device, a multi-vital sign monitoring device, or the like. The ECG device may receive cardiac electrical signals via the plurality of electrodes 128, and may generate the ECG tracing based on the cardiac electrical signals. The ECG may be a single-lead ECG, a 3-lead ECG, a 5-lead ECG, a 6-ead ECG, a 12-lead ECT, or the like. The ECG device 110 may include any number of electrodes 128. For example, the ECG device 110 may include electrodes for generating a 12-lead ECG. In this example, the leads may include leads I, II, III, aVF, aVR, aVL, V1, V2, V3, V4, V5, and V6. The ECG device 110 may be configured to use a subset of the standard 12 leads, or alternatively an ECG using non-standard lead placements (e.g., such as Holter monitor lead placements) or synthesized ECG leads using the actual acquired lead set (e.g., such as GE HealthCare's 12RL algorithm used to synthesize a 12 lead ECG from a reduced lead set).

The ECG analysis device 112 may be configured to receive the ECG from the ECG device 110 and output a diagnosis using the AI-based ECG analysis model 114. In some examples, the AI-based ECG analysis model 114 is a machine learning model. For example, the AI-based ECG analysis model 114 may be a deep neural network (DNN), a convolutional neural network (CNN), a recurrent neural network (RNN), or other type of machine learning model. The AI-based ECG analysis model 114 may be trained on a set of training data to generate and output a diagnosis based on identified features of an ingested ECG. In some examples the training data used to train the AI-based ECG analysis model 114 may be unknown. In other examples, the training data used to train the AI-based ECG analysis model 114 may be known, for example when the AI-based ECG analysis model 114 is generated in-house.

The interpretability and bias module 116 may include instructions therein for adding interpretability to the AI-based ECG analysis model 114, based on interpretable criteria determined using the platform 118 and AI model 120. Further the interpretability and bias module 116 may include instructions therein for assessing bias of outputs of the AI-based ECG analysis model 114. In some example, bias assessment may be executed in part by subject-matter experts.

The platform 118 may be configured to extract, using the AI model 120, interpretable criteria for determining a diagnosis by the AI-based ECG analysis model 114. For example, the platform 118 may be a server, a cloud computing system, or the like. The interpretable criteria that are extracted may include one or both of single-ECG criteria and longitudinal criteria.

The AI model 120 may be configured to determine interpretable criteria for determining a diagnosis by the AI-based ECG analysis model 114. For example, the AI model 120 may be a decision tree (e.g., a classification tree, a regression tree, or the like), a linear regression model, a neural network (e.g., a deep neural network (DNN), a convolutional neural network (CNN), a recurrent neural network (RNN), or the like), a logistic regression model, a support vector machine, or the like.

The user device 122 may be configured to receive the criteria for determining a diagnosis by the AI-based ECG analysis model 114, and provide the criteria for determining a diagnosis by the AI-based ECG analysis model 114 for display. For example, the user device 122 may be a smartphone, a laptop computer, a desktop computer, a wearable device, a medical device, or the like. The user device 122 may be configured to display the diagnosis determined by the AI-based ECG analysis model 114, as well as characteristics of the model (e.g., prediction scores) and bias assessments.

The database 124 may be configured to store an ECG, a set of features of the ECG, a diagnosis of the ECG, a diagnostic interpretation of the ECG, modification information, patient information associated with the ECG, a classification target of the ECG, or the like. Further, the database 124 may be configured to store criteria for determining a diagnosis by the AI-based ECG analysis model 114, including single-ECG criteria and/or longitudinal criteria. For example, the database 124 may be a hierarchical database, a network database, a relational database, or the like. Criteria stored in the database 124 may be initially generated by a subject-matter expert, in some examples. For example, the database 124 may be a database of the Marquette^{™} 12SL ECG analysis program, or other similar program.

The one or more medical data repositories 130 may include one or more of an electronic medical record (EMR) database, an ECG database, a radiology information system (RIS), a picture archiving and communication system (PACS), or other typed of database configured to store medical records of a plurality of patients. For example, ECGs performed for a patient may be stored in an ECG database with associated data including single-ECG features, corresponding diagnostic interpretation, a date of acquisition, an ordering provider, and the like.

The network 126 may be configured to permit communication between the devices of the system 100. For example, the network 126 may be a cellular network (e.g., a fifth generation (5G) network, a long-term evolution (LTE) network, a third generation (3G) network, a code division multiple access (CDMA) network, etc.), a public land mobile network (PLMN), a local area network (LAN), a wide area network (WAN), a metropolitan area network (MAN), a telephone network (e.g., the Public Switched Telephone Network PSTN)), a private network, an ad hoc network, an intranet, the Internet, a fiber optic-based network, or the like, and/or a combination of these or other types of networks.

As an example, the interpretability and bias module 116 may communicate with the ECG analysis device 112 to obtain outputs thereof over the network 126. Further, the ECG analysis device 112 may access data from the database 124 and/or the one or more medical data repositories 130 over the network 126. In this way, the system 100 may be an interconnected system by which the modules thereof communicate with each other to execute various processes and/or methods.

The number and arrangement of the devices of the system 100 shown in FIG. 1 are provided as an example. In practice, the system 100 may include additional devices, fewer devices, different devices, or differently arranged devices than those shown in FIG. 1. Additionally or alternatively, a set of devices (e.g., one or more devices) of the system 100 may perform one or more functions described as being performed by another set of devices of the system 100.

FIG. 2 shows a diagram of example components of a device 200 of the example ECG analysis system 100 shown in FIG. 1. The device 200 may correspond to the ECG device 110, the ECG analysis device 112, the platform 118, the user device 122, the interpretability and bias module 116, and/or the database 124. As shown in FIG. 2, the device 200 may include a bus 210, a processor 220, a memory 230, a storage component 240, an input component 250, an output component 260, and a communication interface 270.

The bus 210 may include a component that permits communication among the components of the device 200. The processor 220 may be implemented in hardware, firmware, or a combination of hardware and software. The processor 220 may be a central processing unit (CPU), a graphics processing unit (GPU), an accelerated processing unit (APU), a microprocessor, a microcontroller, a digital signal processor (DSP), a field-programmable gate array (FPGA), an application-specific integrated circuit (ASIC), or another type of processing component. The processor 220 may include one or more processors capable of being programmed to perform a function. Specifically, the processor 220 may include one or more processors 220 configured to perform the operations described herein. Alternatively, multiple processors 220, collectively, may be configured to perform the operations described herein, and each of the multiple processors 220 may be configured to perform a subset of the operations described herein. For example, a first processor 220 may perform a first subset of the operations described herein, a second processors 220 may be configured to perform a second subset of the operations described herein, etc.

The memory 230 may include non-transitory memory, random access memory (RAM), a read only memory (ROM), and/or another type of dynamic or static storage device (e.g., a flash memory, a magnetic memory, and/or an optical memory) that stores information and/or instructions for use by the processor 220. For example, the memory 230 may store executable instructions thereon that are executable by the processor 220 to perform the operation described herein.

The storage component 240 may store information and/or software related to the operation and use of the device 200. For example, the storage component 240 may include a hard disk (e.g., a magnetic disk, an optical disk, a magneto-optic disk, and/or a solid state disk), a compact disc (CD), a digital versatile disc (DVD), a floppy disk, a cartridge, a magnetic tape, and/or another type of non-transitory computer-readable medium, along with a corresponding drive.

The input component 250 may include a component that permits the device 200 to receive information, such as via user input (e.g., a touch screen display, a keyboard, a keypad, a mouse, a button, a switch, a camera, and/or a microphone for receiving the reference audio input and/or visual input). Additionally or alternatively, the input component 250 may include a sensor for sensing information (e.g., a global positioning system (GPS) component, an accelerometer, a gyroscope, and/or an actuator). The output component 260 may include a component that provides output information from the device 200 (e.g., a display, a speaker for outputting sound at the output sound level, and/or one or more light emitting diodes (LEDs)).

The communication interface 270 may include a transceiver-like component (e.g., a transceiver and/or a separate receiver and transmitter) that enables the device 200 to communicate with other devices, such as via a wired connection, a wireless connection, or a combination of wired and wireless connections. The communication interface 270 may permit the device 200 to receive information from another device and/or transmit information to another device. For example, the communication interface 270 may include an Ethernet interface, an optical interface, a coaxial interface, an infrared interface, a radio frequency (RF) interface, a universal serial bus (USB) interface, a Wi-Fi interface, a cellular network interface, or the like.

The device 200 may perform one or more processors and methods herein described. The device 200 may perform these processes and/or methods based on the processor 220 executing software instructions stored in a non-transitory computer-readable medium, such as the memory 230 and/or the storage component 240. A computer-readable medium may be defined herein as a non-transitory memory device. A memory device may include memory space within a single physical storage device or memory space spread across multiple physical storage devices.

The software instructions may be read into the memory 230 and/or the storage component 240 from another computer-readable medium or from another device via the communication interface 270. When executed, the software instructions stored in the memory 230 and/or the storage component 240 may cause the processor 220 to perform one or more processes and/or methods described herein. Additionally or alternatively, hardwired circuitry may be used in place of or in combination with software instructions to perform one or more methods and/or processes described herein. Thus, implementations described herein are not limited to any specific combination of hardware circuitry and software.

The number and arrangement of the components shown in FIG.2 are provided as an example. In practice, the device 200 may include additional components, fewer components, different components, or differently arranged components than those shown in FIG. 2. Additionally or alternatively, a set of components (e.g., one or more components) of the device 200 may perform one or more functions described as being performed by another set of components of the device 200.

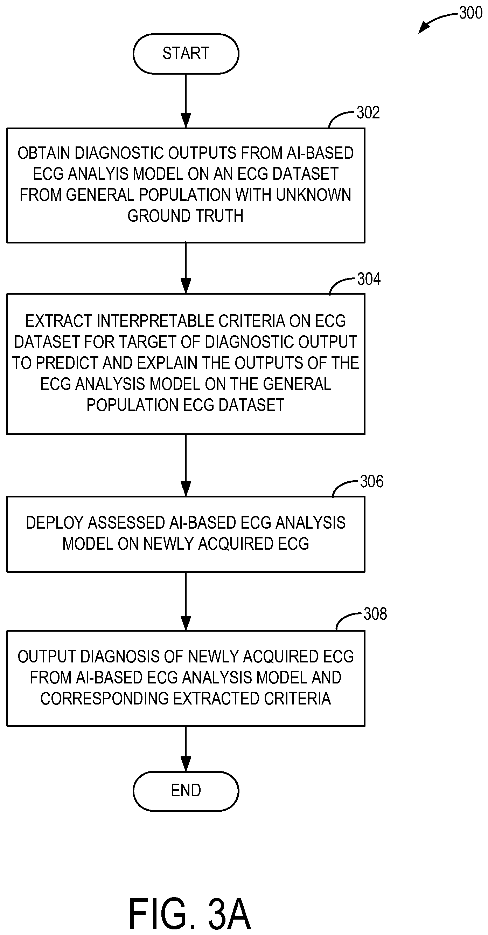

Referring now to FIG. 3A, a flowchart illustrating a method 300 for adding interpretability to and assessing bias for an AI-based ECG analysis model is shown. For example, the AI-based ECG analysis model may be the AI-based ECG analysis model 114 described with respect to FIG. 1. Thus, the AI-based ECG analysis model may be a DNN, CNN, or other type of machine learning model. The method 300 may be executed by one or more processors (e.g., processor 220) according to instructions stored in non-transitory memory (e.g., memory 230).

At 302, method 300 includes obtaining diagnostic outputs from the AI-based ECG analysis model on an ECG dataset from a general population with unknown ground truth. The diagnostic outputs may be obtained during a development phase of the model, whereby the diagnostic outputs are exemplary outputs given for assessment of the model before the model is actually deployed. The ECG dataset may include one or more ECGs that are acquired from the general population (e.g., a population of patients with a wide variety of diagnoses, medical histories, and ECG features). The AI-based ECG analysis model may output a diagnosis and/or diagnostic interpretation for each of the ECGs in the ECG dataset. Thus, the obtained diagnostic outputs may comprise an output for each ECG in the ECG dataset.

Each ECG in the ECG dataset may comprise a set of features (e.g., ECG features) therein. For example, the set of features may include an amplitude of a P wave, a duration of the P wave, an amplitude of a Q wave, a duration of the Q wave, an amplitude of an R wave, a duration of the R wave, a PR interval, an amplitude of an S wave, a duration of the S wave, a duration of the QRS complex, an amplitude of a T wave, a duration of the T wave, a QT interval, or the like. Additionally, or alternatively, the set of features may be defined by the data from a single lead (e.g., single lead specific P wave amplitude or P wave duration), or by data from multiple leads (e.g., global QT duration from earliest Q onset to latest T offset across all leads, or QT dispersion, which is a difference between shortest and longest single lead QT interval measurement across all leads), or by data from spatial relationships across multiple leads (e.g., spatial QRS-T angle or ventricular gradient).

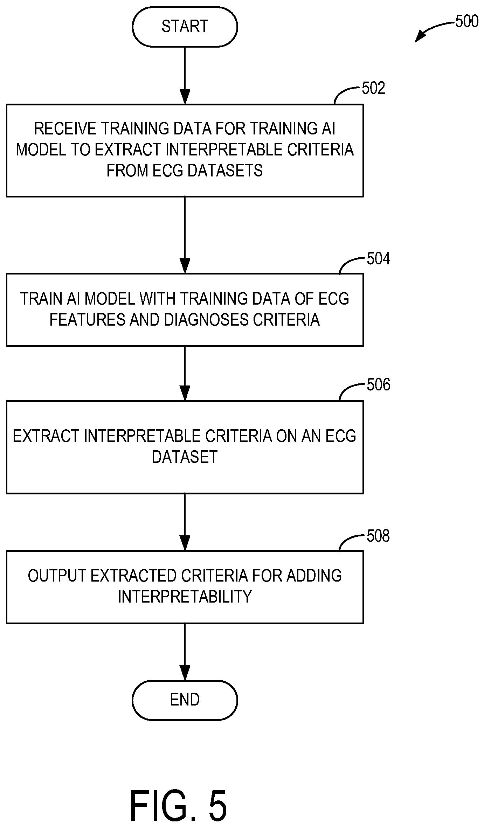

At 304, method 300 includes extracting interpretable criteria on the ECG dataset for a target of the diagnostic output from the AI-based ECG analysis model to predict and explain the diagnostic outputs of the AI-based ECG analysis model. An exemplary method for extracting criteria is described further with respect to FIG. 5. The criteria extracted may include combinations of ECG features (e.g., parameters) and/or sub-combinations of ECG features that together lead to an interpretation endpoint. As noted, the interpretation endpoints may correspond to the diagnostic outputs of the ECG analysis model. The interpretation endpoint may be a diagnostic interpretation, a diagnosis, or the like.

In some examples, one or more characteristics of the extracted criteria may also be determined that give further context to the diagnostic output of the AI-based ECG analysis model. For example, predictive value of each of the criteria may be determined. In some examples, criteria with high positive or negative predictive values may be provided for any ECG acquired in the future as explanatory context for the ECG analysis model output. Extracting criteria and identifying criteria with high predictive values may thus add interpretability to a neural network such as the AI-based ECG analysis model.

At 306, method 300 includes deploying the assessed AI-based ECG analysis model. As noted, interpretable criteria are extracted during the development phase for assessment of the AI-based ECG analysis model. Once assessment is performed, the model may be deployed on a newly acquired ECG. The newly acquired ECG may be an ECG acquired directly from an ECG device (e.g., ECG device 110 of FIG. 1), or an ECG acquired from a medical data repository, such as an ECG database (e.g., one of the one or more medical data repositories 130 of FIG. 1).

At 308, method 300 includes outputting a diagnosis for the newly acquired ECG from the assessed AI-based ECG analysis model and corresponding interpretable criteria to a user device. As an example, a portion of the extracted interpretable criteria that may apply to the newly acquired ECG may be determined based on features of the newly acquired ECG or based on the output of the AI-based ECG analysis model. The diagnosis and the corresponding interpretable criteria may be outputted to the user device 122 described with respect to FIG. 1 that is communicably coupled to the AI-based ECG analysis model, the ECG device, and the module configured with instructions for adding interpretability as in the method 300 herein described. As an example, the interpretable criteria may be displayed on the user device in a list, chart, panel, or the like along with the diagnostic output of the AI-based ECG analysis model. In this way, the user may view both the displayed interpretable criteria alongside the displayed diagnostic output from the AI-based ECG analysis model and in some examples the ECG tracing itself, allowing for easy review and comparison therebetween for increased understanding of the diagnostic output.

In this way, interpretability may be added to an AI-based ECG analysis model when the ground truth of the ECG dataset is unknown by extracting criteria of the ECG dataset during development and assessment of the model. Thus, when deployed for an ECG, the interpretable criteria may also be outputted in order to explain the output of the AI-based model. For example, the criteria may be provided as explainable context at time of deployment, so that a user can better understand and act on the AI-based ECG analysis model's diagnosis. Further, based on the outputs and the predicted classification outputs generated based on the extracted criteria, bias may be assessed for, as will be described below.

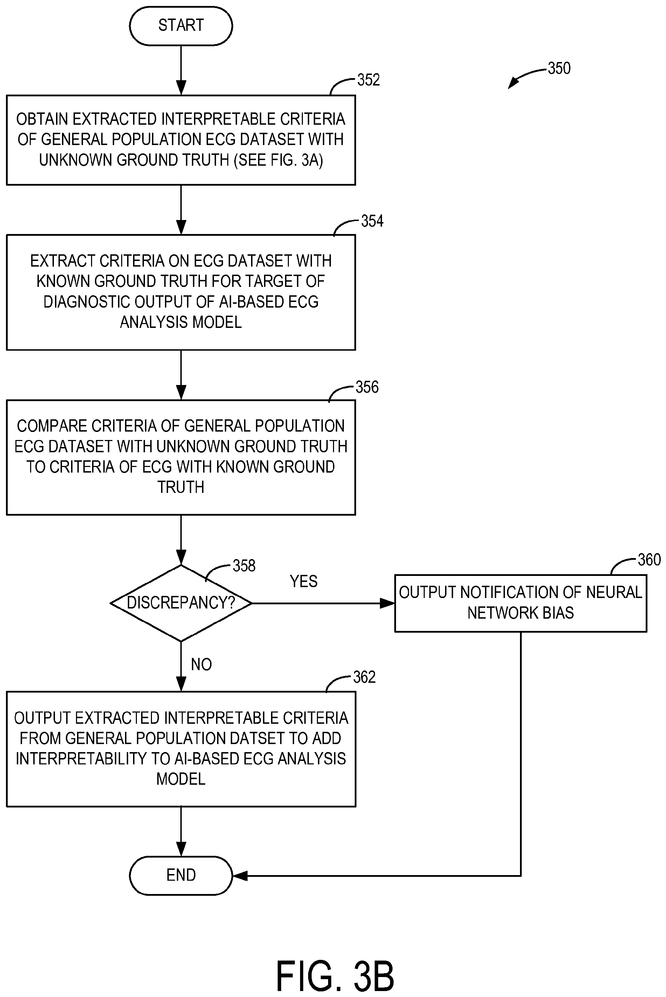

FIG. 3B shows a flowchart illustrating a method 350 for assessing for bias in an AI-based ECG analysis model. For example, the AI-based ECG analysis model may be the AI-based ECG analysis model 114 described with respect to FIG. 1. Thus, the AI-based ECG analysis model may be a DNN, CNN, or other type of machine learning model. The method 300 may be executed by one or more processors (e.g., processor 220) according to instructions stored in non-transitory memory (e.g., memory 230). The method 350 may be executed during a development phase for the AI-based ECG analysis model, in some examples.

At 352, method 350 comprises obtaining extracted interpretable criteria of a general population ECG dataset with unknown ground truth. As described with respect to method 300 above, diagnostic outputs of the AI-based ECG analysis model on the general population ECG dataset with unknown ground truth may be obtained. Using the diagnostic outputs as targets, the interpretable criteria of the general population ECG dataset may be determined. Interpretable criteria may be extracted from ECG dataset with known ground truth based on the diagnostic outputs of the AI-based ECG analysis model (e.g., where the diagnostic outputs of the AI-based ECG analysis model is the target or interpretation endpoint).

At 354, method 350 includes extracting criteria on an ECG dataset with known ground truth for a target of a diagnostic output of the AI-based ECG analysis model. In some examples, the ECG dataset may be a dataset particular to a specific selected population, and the specific selected population may provide an interpretation endpoint, thus giving the ground truth of the dataset. The interpretation endpoints of such a selected population may be confirmed via other procedures, such as echocardiography, in some examples. In other examples, the ECG dataset may be a general population dataset for simple or common endpoints (e.g., interpretation endpoints commonly seen among a general population) for which the ground truth may be taken from interpretation by expert over-readers. In this instance, predicting the ground truth may refer to the process of extracting interpretable criteria that predict and/or characterize the ground truth. That set of criteria can be contrasted to the criteria that characterize the prediction of the AI-based ECG analysis model on a general population ECG dataset (e.g., the interpretable criteria extracted in method 300), as will be described below. Any bias due to overfitting may be determined based on this comparison.

At 356, method 350 includes comparing the extracted criteria of the general population ECG dataset with unknown ground truth, as obtained at 352, to the extracted criteria from the ECG dataset with known ground truth. At 358, method 350 may then include determining if there is a discrepancy between the extracted criteria from the general population ECG dataset with unknown ground truth and the extracted criteria from the ECG dataset with known ground truth. A discrepancy therebetween may indicate presence of bias in the neural network. For example, an ML-based process may be trained on some target, to extract interpretable criteria (e.g., ECG patterns) that characterize that target. The target may be the ground truth of the ECG dataset, or it may be the AI model's predictions. The comparison between the two sets of extracted criteria can be a very deep probe for potential bias in the AI-based ECG analysis model. If there is a discrepancy (YES at 358), method 350 proceeds to 360 to output a notification to the user, for example via a user device (e.g., user device 122), indicating presence of neural network bias. The bias may be due to overfitting, distribution shift, or the like. If there is no discrepancy (NO at 358), method 350 proceeds to 362.

At 362, method 350 includes outputting the extracted interpretable criteria from the general population ECG dataset with unknown ground truth to add interpretability to the AI-based ECG analysis model. For example, similar to as described with respect to method 300, the extracted criteria may be used during the development phase of the model and then may be outputted along with a diagnosis from deployment of the AI-based ECG analysis model on a newly acquired ECG. As described with respect to method 300, the interpretable criteria that are determined for the general population ECG dataset to explain the diagnostic output of the model may be outputted to the user device (e.g., user device 122) along with the diagnostic output of the model. The user may thus review the interpretable criteria to be able to interpret and understand the diagnostic output of the AI-based ECG analysis model.

In this way, interpretability may be added to an AI-based ECG analysis model even with unknown ground truth of the ECG dataset. For example, interpretability may be added by extracting criteria of the ECG dataset for which the ECG analysis model is providing output for the given diagnostic output interpretation endpoint during development of the model. Then, once the interpretable criteria are extracted, the model may be deployed and corresponding criteria may be provided to explain the output of the model.

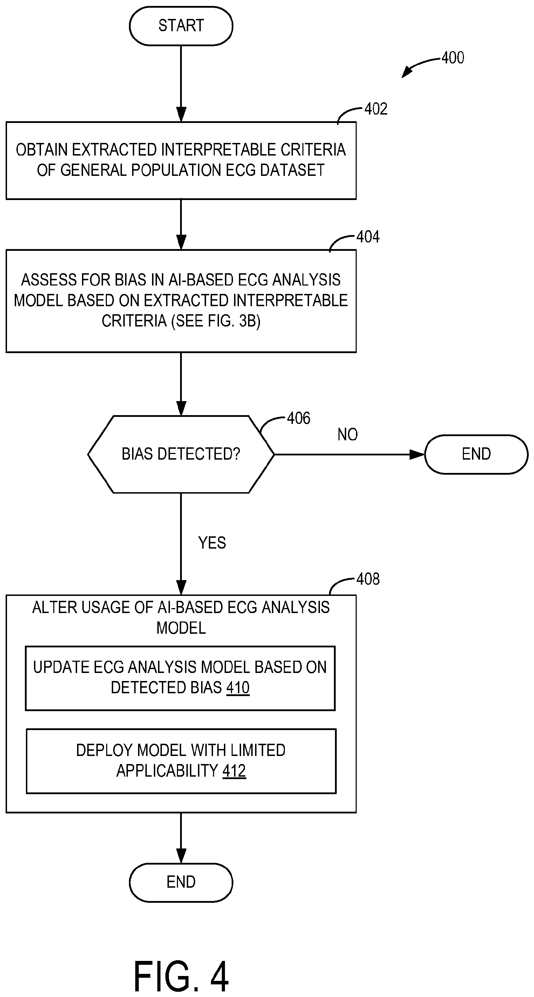

Turning now to FIG. 4, a flowchart illustrating a method 400 for assessing bias in an AI-based ECG analysis model is shown. The method 400 may be performed in conjunction with either the method 300 or the method 350, in some examples, depending on whether the ground truth of the ECG dataset is known. For example, the AI-based ECG analysis model may be the AI-based ECG analysis model 114 described with respect to FIG. 1. Thus, the AI-based ECG analysis model may be a DNN, CNN, or other type of machine learning model. The method 300 may be executed by one or more processors (e.g., processor 220) according to instructions stored in non-transitory memory (e.g., memory 230).

At 402, method 400 includes extracting interpretable criteria on an ECG dataset from a general population. For example, as described with respect to FIG. 3A, diagnostic outputs of the AI-based ECG analysis model on an ECG dataset from the general population may be obtained. In some examples, the ground truth may typically not be available for a dataset from a general population, and thus is not required for the method 400. Thus the general population ECG dataset may be herein referenced as having unknown ground truth. With the diagnostic outputs of the AI-based ECG analysis model as interpretation endpoints, interpretable criteria can be extracted from the ECG dataset, as will be further described with respect to FIG. 5. The interpretable criteria that are extracted may thus explain the diagnostic outputs from the AI-based ECG analysis model.

At 404, method 400 includes assessing for bias in the AI-based ECG analysis model based on the extracted interpretable criteria. The potential of bias by the AI-based ECG analysis model can be assessed from the interpretable criteria that explain its outputs, as explained with respect to FIG. 3B. Other techniques to assess bias may include comparison to expectations from literature as determined by human experts and/or comparison to criteria that explain the outputs of the AI-based ECG analysis model as extracted on the training dataset thereof in examples when the ground truth is known. The capability to assess on a general population ECG dataset may thus access previously undiscovered forms of bias. For example, bias that may arise from distribution shift may be detected with this method. In general, even external testing of AI-based ECG analysis models are performed on a selected population for which the ground truth is known. Previously undiscovered forms of bias may thus be discovered by assessing the outputs of the AI-based ECG analysis model on a general population.

At 406, method 400 determines if bias is detected. Determining if bias is detected may indicate a fitness of the AI-based ECG analysis for deployment and/or indicate to a user whether to trust a provided output. If bias is detected based on the techniques described above, method 400 proceeds to 408. If no bias is detected, method 400 ends. When no bias is detected, there may be a high degree of confidence that the AI-based ECG model can be deployed without bias and that the diagnostic outputs thereof when deployed can be trusted. For example, the interpretable criteria extracted for the AI-based ECG analysis model may be outputted as described above without any changes to the AI-based ECG analysis model or to its outputs.

At 408, when bias is detected, method 400 includes altering usage of the AI-based ECG analysis model. In some examples, altering the usage of the AI-based ECG analysis model may include updating the AI-based ECG analysis model based on the detected bias, as noted at 410. For example, the AI-based ECG analysis model may be returned to a model development phase to reduce the potential of the model from acquiring the detected bias, which may be achieved by training on a dataset that may be more representative of the population that the model is intended to be deployed on. In such examples, the methods herein may be repeated to once again assess for bias to determine if updating the model removed the detected bias. In other examples, altering the usage of the AI-based ECG analysis model may include deploying the AI-based ECG analysis model with limited applicability, as noted at 412. For example, a notification may be outputted along with the interpretable criteria extracted as they relate to the diagnostic outputs indicating that bias was detected and thus the applicability of the diagnostic outputs may be limited. For example, when potential for bias is detected under the presence of specific criteria, then the model output may be suppressed in the presence of those criteria, or a warning that conveys reduced confidence may be displayed as a notification to the user on the user device.

Whether or not bias is detected, the bias assessment may be outputted to the user device. For example, when bias is not detected, a notification that bias is not detected may be displayed on the user device alongside the extracted interpretable criteria. When bias is detected, the bias assessment that indicates presence of bias and/or how the model is updated (e.g., returned to model development or deployed with limited applicability) may be displayed on the user device (e.g., user device 122). In some examples, the amount or level of bias may be displayed as a comparison to a predefined threshold amount of bias. For example, if an amount of bias is detected below a predefined threshold, the bias assessment may be displayed to include the amount of bias as well as the comparison to the predefined threshold. Thus, both the bias assessment as well as the interpretable criteria may be displayed on the user device alongside the diagnostic output of the AI-based ECG analysis model, thus allowing the user to more fully analyze and understand the output of the model.

Turning to FIG. 5, a flowchart illustrating a method 500 for extracting interpretable on an ECG dataset for adding interpretability to an AI-based ECG analysis model (e.g., a DNN, CNN, RNN, or the like). The method 500 may be executed by one or more processors (e.g., processor 220) according to instructions stored in non-transitory memory (e.g., memory 230). It should be appreciated that the method 500 herein described is exemplary in nature, and other methods of extracting interpretable criteria for adding interpretability to the AI-based ECG analysis model may be possible without departing from the scope of this disclosure.

At 502, method 500 includes receiving training data for training an AI model to extract interpretable criteria from ECG datasets. The AI model may be trained to extract interpretable criteria so as to add interpretability to AI-based ECG analysis models as herein disclosed. As an example, a platform, such as platform 118 of the ECG analysis system 100 of FIG. 1, may receive training data for training the AI model, such as the AI model 120 of FIG. 1.

As an example, the training data may include an ECG as an input and criteria thereof as targets. For example, the ECG may be a waveform acquired via the ECG device 110. Additionally or alternatively, the inputs of the training data may include a set of features for the ECG. For example, the set of features may include an amplitude of a P wave, a duration of the P wave, an amplitude of a Q wave, a duration of the Q wave, an amplitude of an R wave, a duration of the R wave, a PR interval, an amplitude of an S wave, a duration of the S wave, a duration of the QRS complex, an amplitude of a T wave, a duration of the T wave, a QT interval, or the like. Additionally or alternatively, the set of features may be defined by the data from a single lead (e.g., single lead specific P wave amplitude or P wave duration), or by data from multiple leads (e.g., global QT duration from earliest Q onset to latest T offset across all leads, or QT dispersion, which is a difference between shortest and longest single lead QT interval measurement across all leads), or by data from spatial relationships across multiple leads (e.g., spatial QRS-T angle or ventricular gradient).

Targets of the training data may include sets of ECG criteria. ECG criteria may comprise combinations of one or more ECG features that correspond to a diagnosis. For example, an input ECG may include a first feature and a second feature and a target criteria may be the combination of the first feature and second feature that together correspond to a diagnosis. So, for example, the AI model may be trained to ingest an ECG, identify the first and second feature, and extract therefrom, the criteria of the combination of the first and second feature.

Thus, the training data may include a diagnosis of the ECG. The diagnosis may be endpoints for extraction. For example, the diagnosis may include atrial-paced rhythm, ventricular-paced rhythm, atrial flutter, ectopic atrial tachycardia, sinus bradycardia, sinus tachycardia, junctional bradycardia, atrial fibrillation, LBBB, LVH, septal infarct, non-ST elevated myocardial infarction, ST elevated myocardial infarction, or the like. Further, the diagnostic interpretation result may be an interpretation result of the ECG determined by a physician. Additionally or alternatively, the diagnostic interpretation result may be a diagnosis by a physician using the ECG alone, or alternatively using another source of clinical information (e.g., high sensitivity troponin levels, cardiac echo measurements, or angiography findings), or a combination of ECG and non-ECG clinical information.

Further, the training data may include patient information associated with the ECG. For example, the patient information may identify whether a specific diagnosis was present in a previous diagnostic interpretation result of the patient, identify whether a physician had previously modified a diagnosis interpretation result, identify demographic information of the patient, identify health conditions (e.g., prior diagnosis, comorbidities, etc.) of the patient, identify medications prescribed to the patient, identify previous procedures performed on the patient, identify previous diagnoses (e.g., resolved diagnoses) of the patient, and the like.

Further, the training data may include a classification target of the ECG. For example, the classification target may be a diagnosis of the ECG, a diagnostic interpretation of the ECG, modification information of the ECG, or the like as herein noted.

At 504, method 500 includes training the AI model with the training data of the ECG features (inputs) and diagnoses (targets). Specifically, the targets are the diagnoses and the diagnostic criteria is what is extracted through the procedure herein described, by optimizing on reaching the diagnostic target. For example, the platform 118 of the ECG analysis system 100 of FIG. 1 may train the AI model 120 based on the training data. Alternatively, a system or device other than the platform 118 may be used to generate and/or train the AI model 120. For example, a system or device may include instructions for generating the AI model 120, and/or instructions for training the AI model 120. The system or device may provide a resulting trained AI model 120 to the platform 118 for use.

In some examples, the AI model 120 may include a training phase, a deployment phase, and a monitoring phase. In the training phase, the platform 118 may receive and process training data to generate the trained AI model 120 for extracting the criteria for explaining a diagnostic criteria by the ECG analysis model. The training data may include a plurality of training datasets respectively including one or more of ECG(s), a set of features of the ECG(s), a diagnosis of the ECG, a diagnostic interpretation of the ECG, modification information, patient information associated with the ECG, a classification target of the ECG, and/or the like. Each training dataset of the plurality of training datasets may be associated with a particular ECG and a particular diagnostic interpretation result.

The training data may be generated, received, or otherwise obtained from internal and/or external resources. For example, the training data may be generated, received, or otherwise obtained from the ECG device 110, the ECG analysis device 112, the user device 122, and/or the database 124.

Generally, the AI model 120 may include a set of variables (e.g., nodes, neurons, filters, or the like) that are tuned (e.g., weighted, biased, or the like) to different values via the application of the training data. According to an embodiment, the training process may employ supervised, unsupervised, semi-supervised, and/or reinforcement learning processes to train the AI model 120. According to an embodiment, a portion of the training data may be withheld during training and/or used to validate the trained AI model 120.

For supervised learning processes, the training data may include labels or scores that may facilitate the training process by providing a ground truth. For example, the labels or scores may indicate a classification target. Training may proceed by feeding a training dataset into the AI model 120. The AI model 120 may have variables set at initialized values (e.g., at random, based on Gaussian noise, based on pre-trained values, or the like). The AI model 120 may generate an output. The output may be compared with the corresponding label or score (e.g., the ground truth), which may then be back-propagated through the AI model 120 to adjust the values of the variables. This process may be repeated for a plurality of samples at least until a determined loss or error is below a predefined threshold. According to an embodiment, some of the training data may be withheld and used to further validate or test the trained AI model 120.

For unsupervised learning processes, the training data may not include pre-assigned labels or scores to aid the learning process. Instead, unsupervised learning processes may include clustering, classification, or the like, to identify naturally occurring patterns in the training data. As an example, training data may be clustered into groups based on identified similarities and/or patterns. K-means clustering or K-Nearest Neighbors may also be used, which may be supervised or unsupervised. Combinations of K-Nearest Neighbors and an unsupervised cluster technique may also be used. For semi-supervised learning, a combination of training data with pre-assigned labels or scores and training data without pre-assigned labels or scores may be used to train the AI model 120.

When reinforcement learning is employed, an agent (e.g., an algorithm) may be trained to make a decision regarding whether a diagnostic interpretation should be modified from the training data through trial and error. For example, based on making a decision, the agent may then receive feedback (e.g., a positive reward if the prediction was above a predetermined threshold), adjust its next decision to maximize the reward, and repeat until a loss function is optimized.

After being trained, the trained AI model 120 may be stored and subsequently applied by the platform 118 during the deployment phase. For example, during the deployment phase, the trained AI model 120 executed by the platform 118 may receive input data, and generate output data. During a monitoring phase, monitoring data may be analyzed along with the output data and input data to determine an accuracy of the trained AI model 120. According to an embodiment, based on the analysis, the platform 118 may return to the training phase, where values of one or more variables of the AI model 120 may be adjusted to improve the accuracy of the AI model 120.

According to an embodiment, the AI model 120 may be a decision tree. In this case, the platform 118 may generate the decision tree using a training technique. For example, the training technique may include a random forest technique, a boosted trees technique, a bootstrap technique, a rotation forest technique, or the like. The AI model 120 may include a set of nodes. For example, the set of nodes may include a root node, one or more intermediate nodes, and leaf nodes. The platform 118 may generate the AI model 120 using an attribute selection measure. For example, the attribute selection measure may be information gain, a gain ratio, a Gini index, or the like. The platform 118 may generate the decision tree, and prune the decision tree using a pruning technique. For example, the pruning technique may be cost complexity pruning, reduced error pruning, or the like.

At 506, method 500 includes extracting interpretable criteria from a general population ECG dataset. For example, the platform 118 of FIG. 1 may extract, using the AI model 120, the criteria for determining the diagnosis by the AI-based ECG analysis model 114. The general population ECG dataset may be the dataset on which the AI-based ECG analysis model is deployed, in some examples. The interpretable criteria may be extracted with respect to a diagnostic output of the AI-based ECG analysis model. For example, the diagnostic outputs of the AI-based ECG analysis model on the general population ECG dataset may be obtained and then used as the interpretation endpoint when extracting the interpretable criteria.

As herein described, the platform 118 may determine a decision branch of the AI model 120 that corresponds to a particular target classification. The particular target classification in this example may be the diagnostic output of the AI-based ECG analysis model. For example, the target classification may be a diagnosis of the ECG, a diagnostic interpretation result of the ECG, modification information of the ECG, or the like. The decision branch may include one or more nodes corresponding to respective criteria. For example, the decision branch may include a root node, one or more intermediate nodes, and a leaf node.

Further, in some examples, the platform 118 may determine a decision branch of the AI model 120 that includes one or more nodes associated with an attribute selection measure that satisfies a threshold. For example, the attribute selection measure may be information gain, a gain ratio, a Gini index, or the like. As a particular example, the platform 118 may determine a decision branch that includes a leaf node corresponding to a criterion having a Gini index that is less than a threshold. According to an embodiment, the platform 118 may determine a decision branch based on a metric of the decision path. For example, the metric may be an accuracy, a positive prediction value, a sensitivity, or the like. Additionally, or alternatively, the platform 118 may determine a decision branch based on a performance on generalization to external datasets.

According to one or more examples, the platform 118 may determine particular nodes of the one or more nodes to use for criteria extraction. The platform 118 may determine the particular nodes based on a feature selection measure. The feature selection measure may correspond to an importance of a feature associated with a criterion corresponding to the node. According to an embodiment, the platform 118 may fit another AI model 120 (e.g., a decision tree) to a dataset that is separate from the training dataset. In this way, the platform 118 may reduce the risk of overfitting, and increase the statistical power of the technique.

In some examples, the platform 118 may extract the criteria from the decision branch. For example, the platform 118 may extract the criteria that belong to the decision branch. Each criterion may include a respective feature and a respective threshold. In some examples, the platform 118 may determine a threshold of a criterion. For example, the platform 118 may determine a threshold based on an optimization technique, based on an input from the user device 122, or the like.

Further, the platform 118 may generate a rule including a set of criteria that were extracted. For example, the rule may include criteria from a particular decision branch. Alternatively, the rule may include criteria from different decision branches. The platform 118 may generate the rule using a permutation and/or combination of criteria. According to an embodiment, the platform 118 may determine a metric of the rule. For example, the metric may be an accuracy, a positive prediction value, a sensitivity, or the like. Further, the platform 118 may determine that the rule includes a metric that satisfies a threshold.

Further, in some examples, predictive value of each of the extracted criteria may also be determined that give further context to the diagnostic output of the AI-based ECG analysis model. In some examples, as previously described criteria with high positive or negative predictive values may be provided for any ECG acquired in the future as explanatory context for the ECG analysis model output. Extracting criteria and identifying criteria with high predictive values may thus add interpretability to a neural network such as the AI-based ECG analysis model.

At 508, method 500 includes outputting the extracted interpretable criteria for adding interpretability to the AI-based ECG analysis model. As described above, the extracted interpretable criteria may be used to explain outputs of the AI-based ECG analysis model, when the outputs are generated on the same dataset for which the criteria are extracted. As an example, the criteria extraction as herein presented may be incorporated into a method for adding interpretability and assessing bias (e.g., into the methods 300, 350, and 400 presented above) during a development phase of the AI-based ECG analysis model.

It should be appreciated that method 500 is herein provided as an example for how to extract criteria and other methods may be performed without departing from the scope of this disclosure.

Turning now to FIG. 6, a diagram 600 of example criteria for determining that may be outputted so as to add interpretability to an AI-based ECG analysis model is shown. As shown in FIG. 6, the platform 118 may generate a first group 610 of criteria, a second group 620 of criteria, a third group 630 of criteria, a fourth group 640 of criteria, and a fifth group 650 of criteria. The platform 118 may generate the groups of criteria based on extracting the criteria from respective decision branches of the AI model 120. The platform 118 may group the criteria based on some commonality or similarity of the criteria, and may generate the groups based on grouping the criteria.

As an example, the first group 610 may include criteria corresponding to non-monomorphic R wave in lateral leads, including peak amplitudes of S waves, peak amplitudes in leads 5 and 6, and the like. The second group 620 may include criteria corresponding to Q waves in lateral leads, including Q wave peak amplitudes, Q wave durations, and the like. The third group 630 may include criteria corresponding to short R wave peak times in lateral leads, including peak times of R waves in leads 5 and 6. The fourth group 640 may include criteria corresponding to lack of appropriate QRS-ST discordance, including ST voltage greater than a threshold in various leads and the like. The fifth group 650 may include criteria corresponding to limited QRS durations, including QRS duration, R wave duration, S wave duration, and the like. Thus, grouping of criteria may be performed based on an overarching theme. Criteria may then be assigned to a group based on satisfied themes.

FIG. 7 shows a graph 700 of prediction scores of an AI-based ECG analysis model, such as the AI-based ECG analysis model 114. A first histogram 704 shows the model prediction score on a large general population. Most samples fall below a threshold prediction score 702, resulting in a negative finding for the searched endpoint. A second histogram 706 shows the model prediction score in the presence of some combination of identified criteria. The area of the second histogram 706 as shown in normalized to be compared directly to the first histogram 704.

When the identified criteria are met, the AI-based ECG analysis model prediction scores may be above the threshold prediction score 702. Thus, for those samples and for further ones where those criteria are met, the criteria are more likely to predict and explain the model's classification output. Thus, criteria with high predictive values may be provided as explanatory context for the model in the future.

Turning now to FIG. 8, a graph 800 of rate of diagnostic output by an AI-based ECG analysis model is shown. Bias assessment may be performed on outputs of the ECG analysis model by parsing identified criteria and their combinations for indications of spuriousness. In the example shown in FIG. 8, the ECG analysis model may output positive predictions more frequently in the presence of a first diagnosis (e.g., atrial fibrillation) and a second diagnosis (e.g., paced rhythm). For example, a first count 802 for the first diagnosis may correspond to negative predictions and a second count 804 may correspond to positive predictions for the first diagnosis. Similarly, a first count 806 may correspond to negative predictions and a second count 808 may correspond to positive predictions. The counts corresponding to positive predictions may be considerably higher than the counts corresponding to negative predictions.

This finding of increased rate of positive predictions in the presence of these diagnoses may indicate bias in the AI-based ECG analysis model. For example, positive prediction rate may be higher in the presence of the diagnoses, which indicates that the model associates those diagnoses with the target endpoint, but for the specific endpoint targeted by the AI-based ECG analysis model, the correlation may be spurious or expected to be different between a training dataset and a general population.

The technical effect of the herein disclosed systems and methods is that interpretability may be added to AI-based ECG analysis models, even when the ground truth on which the ECG dataset on which the AI-based ECG analysis model was deployed on is unknown. Adding interpretability may increase the usability of AI-based ECG analysis models in clinical settings as users may more readily understand why the model outputs the diagnoses and diagnostic interpretations it does. Further, bias may be assessed for and if present, users may be informed that bias in the AI-based ECG analysis model is present, allowing for more accurate assessment of the outputs by the user.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A method for adding interpretability to an artificial intelligence (AI)-based electrocardiogram (ECG) analysis model, comprising:
obtaining diagnostic outputs from the AI-based ECG analysis model on an ECG dataset with unknown ground truth (302);
extracting interpretable criteria from the ECG dataset with interpretation endpoints of the diagnostic outputs from the AI-based ECG analysis model (304);
deploying the AI-based ECG analysis model on an ECG (306); and
outputting a diagnostic output of the AI-based ECG analysis for the ECG and interpretable criteria of the extracted interpretable criteria that correspond to the ECG to a user device, wherein the interpretable criteria explain the diagnostic output (308).

2. The method of claim 1, wherein the AI-based ECG analysis model is a machine learning model (114).

3. The method of claim 1, wherein assessing the AI-based ECG analysis model for bias includes:
extracting criteria from an ECG dataset with known ground truth for an interpretation endpoint of a diagnostic output from the AI-based ECG analysis model for the ECG dataset (354);
comparing the extracted criteria from the ECG dataset with known ground truth to the extracted interpretable criteria from the ECG dataset with unknown ground truth (356); and
determining a discrepancy between the extracted criteria from the ECG dataset with known ground truth and the extracted interpretable criteria from the ECG dataset with unknown ground truth (358).

4. The method of claim 3, wherein the ECG dataset with known ground truth is an ECG dataset of a selected population (354).

5. The method of claim 1, wherein the ECG dataset with unknown ground truth is a general population ECG dataset (302).

6. The method of claim 1, further comprising determining presence of bias in the AI-based ECG analysis model and in response to presence of bias being detected, altering usage of the AI-based ECG analysis model (408), wherein altering usage includes one or more of deploying the AI-based ECG analysis model with limited applicability (412) and returning the AI-based ECG analysis model to a development phase to address the bias (410).

7. The method of claim 1, wherein extracting the interpretable criteria further comprises determining predictive value of each of the interpretable criteria and outputting the extracted interpretable criteria comprises outputting criteria with high predictive value (304).

8. The method of claim 7, wherein criteria with high predictive value are outputted as explanatory context for the diagnostic output of the AI-based ECG analysis model (308).

9. A device, comprising:
a memory (230) configured to store instructions; and
one or more processors (220) configured to, based on the instructions stored in memory:
during a development phase of an artificial intelligence (AI)-based electrocardiogram (ECG) analysis model, obtain a diagnostic output from the AI-based ECG analysis model for a general population ECG dataset (302);
extract interpretable criteria from the general population ECG dataset for a target of the diagnostic output to generate a predicted output of the AI-based ECG analysis model based on the extracted interpretable criteria (304);
determine presence of bias in the AI-based ECG analysis model based on the extracted interpretable criteria (404);
during a deployment phase of the AI-based ECG analysis model, determine a diagnosis of a newly acquired ECG by the AI-based ECG analysis model (306); and
output the diagnosis, the extracted interpretable criteria that explain the outputted diagnosis, and the determined bias to a user device communicatively coupled to the device (308).

10. The device of claim 9, wherein the newly acquired ECG is obtained from an ECG device (110) communicatively coupled to the device.

11. The device of claim 9, wherein presence of bias is determined by comparison to expectations from literature (404).

12. The device of claim 9, wherein, to determine presence of bias, the one or more processors are further configured to:
extract criteria from an ECG dataset with known ground truth for an interpretation endpoint of a diagnostic output from the AI-based ECG analysis model for the ECG dataset with known ground truth (354);
compare the extracted criteria from the ECG dataset with known ground truth to the extracted interpretable criteria from the general population ECG dataset (356); and
determine a discrepancy between the extracted criteria from the ECG dataset with known ground truth and the extracted interpretable criteria from the ECG dataset with unknown ground truth (358).

13. The device of claim 12, wherein, when a discrepancy between the extracted criteria from the ECG dataset with known ground truth and the extracted interpretable criteria from the general population ECG dataset is determined based on the comparison, the one or more processors are further configured to output a notification of bias to the user device, wherein bias is due to one of overfitting and distribution shift (360).

14. The device of claim 9, wherein, in response to detection of presence of bias, one or more processors configured to, based on the instructions stored in memory, alter usage of the AI-based ECG analysis model (408), wherein altering usage includes one or more of deploying the AI-based ECG analysis model with limited applicability (412) and returning the AI-based ECG analysis model to the development phase to address the bias (410).

15. The device of claim 9, wherein the AI-based ECG analysis model (114) is one of a deep neural network (DNN), a convolutional neural network (CNN), and a recurrent neural network (RNN).
